# EUROPEAN PATENT APPLICATION

(11) **EP 4 759 365 A1**
(43) Date of publication of application: **17.06.2026**
(21) Application number: 25193941.9
(22) Date of filing: 05.08.2025
(51) Int. Cl.: A61P 27/02, A61P 35/00, A61P 43/00, C07K 16/22

(54) **NEW FUSION PROTEIN AGAINST ANGIOGENESIS**

(30) Priority: 13.12.2024 US 202418980371
(71) Applicant: Trican Biotechnology Co., Ltd., New Taipei City 221 (TW)
(72) Inventor: WANG, Ya-Hsuan, New Taipei City (TW); LIN, Shin-Chang, New Taipei City (TW); LI, Cheng-Ke, New Taipei City (TW); CHIU, Shih-Han, New Taipei City (TW); KAO, Pu-Hung, New Taipei City (TW); SHEN, San-Tai, New Taipei City (TW)
(74) Representative: Straus, Alexander

(57) **Abstract**

The present invention provides a new bi-functional fusion protein TC1545, simultaneously targeting vascular endothelial growth factor (VEGF) and angiopoietins (ANGs) with a higher monomer purity. The bi-functional fusion protein contains two or more domains of human proteins and are of all human sequences, which can be used therapeutically in human targeting angiogenesis-associated diseases.

## Description

### FIELD OF THE INVETNTION

The present invention relates to a bi-functional fusion protein, which comprises two binding moieties including a fragment antigen-binding (Fab) region and a single-chain fragment variable (scFv) region of antibodies for treating angiogenesis.

### BACKGROUD OF THE INVENTION

Angiogenesis has been reported as a key mechanism in the disease progress of neovascular age-related macular degeneration (AMD). The generation of new capillaries in choroid destroys and penetrates the Bruch's membrane to reach the subretinal pigment epithelial space. The penetrated choroidal new vessels are leaky and unbalance the nutrient and waste transport in the local region of retina, especially the retinal pigment epithelial cells and photoreceptor. The imbalance of the system causes the retina to swell and affects the photoreceptor's normal function, leading to loss of vision eventually.

Vascular endothelial growth factor (VEGF) and angiopoietins (ANGs) both play important roles in the process of angiogenesis. The VEGF-A has been shown to stimulate endothelial cell mitogenesis, cell survival and proliferation, and increase microvascular permeability. Studies have exhibited the therapeutic effectiveness by blocking VEGF activity with antibodies, VEGF traps or VEGFR inhibitors on cancer and AMD treatment. ANG2 was also reported to induce endothelial destabilization, vessel regression and hypoxia through ANG-TIE pathway. Both VEGF and ANG2 has been demonstrated to induce strong angiogenesis in the tumor. Therefore, combination of VEGF-A and ANG2 blocker has become a promising strategy for cancer and AMD treatment.

In addition to the fundamental mechanism of therapeutic antibodies or blockers, their manufacturing feasibility and stability would be another critical consideration on the drug development. For example, antibody aggregation in therapeutic treatment may cause severe immunogenicity issues, such as allergic response and reduce therapeutic efficacy by inducing anti-drug antibodies (ADAs) to enhance drug clearance. To ensure the therapeutic antibody with high purity and stability is the essential criteria during the drug development. Currently, the administration of neovascular AMD therapeutic drugs replies on periodically intravitreal injection with high concentration of fusion protein or bispecific antibody. For example, the recommended therapeutic dose for Eylea is 0.05 mL of fusion protein Eylea at 40 mg/mL concentration (in total 2 mg for each injection) every 4 weeks. The administration dose of Vabysmo, a bispecific antibody approved for neovascular AMD therapy in 2022, is 0.05 mL of Vabysmo at concentration of 120 mg/mL (in total 6 mg per injection) every 4 weeks for the first doses.

Therefore, to make sure whether potential drug candidates can maintain their purity and stability at high protein concentration is a necessary process in the ophthalmic drug development.

Previously, a bi-functional fusion protein, AT-Fc-VT and TC350-253, simultaneously targeting vascular endothelial growth factor (VEGF) and angiopoietins (ANGs), were provided in U.S. Patent Nos. 11,518,819 and 12,054,561, respectively, which were created and applied for patent by Trican Biotechnology Co., Ltd. However, TC350-253 showed instability and aggregation at high concentration on downstream process development, and it is unlikely can launch a clinical study under these instability and aggregation issues.

Accordingly, it is still desirable to develop an engineered fusion protein to improve the stability and maintain its promising bi-functional activity on anti-angiogenesis.

### BRIEF SUMMARY OF THE INVENTION

The present invention provides a fusion protein that inhibits an ANG-TIE signaling pathway and a VEGF-VEGFR signaling pathway, wherein the fusion protein contains an ANG inhibiting domain and a VEGF inhibiting domain thereby providing a significantly improved efficacy in inhibition of angiogenesis through blocking the interactions between ANG, VEGF, or both with their receptors.

In one aspect, the present invention provides a bi-functional fusion protein TC1545, which is composed of (1) a light chain arm having the amino acid sequence set forth in SEQ ID NO: 4 and (2) a heavy chain arm having the amino acid sequence set forth in SEQ ID NO: 7, comprising (a) an antigen-binding (Fab) fragment of anti-ANG2 antibody, (b) a stabilized single-chain fragment variable (scFv) fragment of anti-VEGF antibody, and (c) a linker, wherein the light chain arm and the heavy chain arm is bridged with a disulfide bond.

In a further aspect, the present invention provides a pharmaceutical composition for use in inhibiting angiogenesis, comprising the bi-functional fusion protein TC1545 and a pharmaceutically acceptable carrier.

In one embodiment of the present invention, the light chain arm comprises a variable light chain region (V_{L}) having the amino acid sequence set forth in SEQ ID NO: 2

In one embodiment of the present invention, the heavy chain arm comprises (1) a Fd fragment having the amino acid sequence set forth in SEQ ID NO: 3, (2) a scFv fragment having the amino acid sequence set forth in SEQ ID NO: 6, and (3) a linker having the amino acid sequence set forth in SEQ ID NO: 5.

In one embodiment of the present invention, the antigen-binding (Fab) fragment and the single chain antibody (scFv) fragment are fused with a linker having the amino acid sequence set forth in SEQ ID NO: 5.

In one embodiment of the present invention, the bi-functional fusion protein TC1545 has unexpected modifications to the TC350-253, particularly a five amino acid insertion between residue 229-230, a two amino acid replacement at residue 289 and 483, and a six amino acid deletion of residue 491-496 of TC350 having the amino acid sequence set forth in SEQ ID NO: 8.

In one example of the invention, the antigen-binding (Fab) fragment is an ANG2 binding motif-containing fragment which is composed of the amino acid sequence set forth in SEQ ID NO: 3 and SEQ ID NO: 4.

In one example of the invention, the single chain antibody (scFv) fragment is a VEGF binding motif-containing fragment having the amino acid sequence set forth in SEQ ID NO: 6.

According to the invention, the bi-functional fusion protein TC1545 is effective in preventing or treating an angiogenesis-associated disease, such as age-related macular degeneration (AMD) disease or a primary or metastatic cancer.

In addition, the bi-functional fusion protein TC1545 is effective in inhibiting angiogenesis of cancer cells, and accordingly it can be used in treating a primary or a metastatic cancer.

In a yet aspect, the present invention provides a method for preventing or treating an angiogenesis-associated disease in a subject, comprising administering to said subject the pharmaceutical composition according the invention.

According to the invention, the angiogenesis-associated disease comprises age-related macular degeneration (AMD) disease, primary cancer and metastatic cancer.

According to the invention, the fusion protein TC1545 not only exhibit a higher monomer purity as compared to the fusion protein TC350-253, but also has an efficacy in inhibiting angiogenesis, which is effective for preventing or treating age-related macular degeneration (AMD) disease, or for treating a primary or a metastatic cancer.

It is to be understood that both foregoing general description and the following detail description are exemplary and explanatory and are not restrictive of the invention.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred.

In the drawings:
Figure 1A-1B show the structure diagram of TC1545 (Figure 1A) and TC350-253 (Figure 1B) bi-functional fusion proteins, respectively. Two mutations to form a disulfide-bond were introduced within scFv domain of VEGF binding fragment to stabilize the fusion protein TC1545.

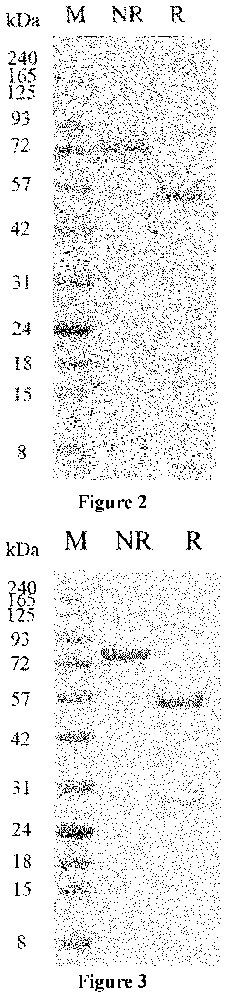

Figure 2 shows the SDS-PAGE analysis of TC1545. Both cDNAs encoded for the heavy-chain arm and light-chain arm polypeptide of TC1545 were constructed into one UCOE-GS vector (Merck) and then over-expressed with CHOZN cell line (Merck). The bi-functional fusion protein TC1545 were purified from the cell supernatant using Mabselect VL resines, ceramic HA affinity chromatography and ionic exchange chromatography. The purified protein (2 µg/lane) were analyzed by SDS-PAGE under reducing (R) or non-reducing (NR) condition. The molecular weight of this bispecific antibody is about 75 kD, and the purity is great than 90%.
Figure 3 shows the SDS-PAGE analysis profile of the bi-functional fusion protein TC350-253. Heavy-chain arm TC350 plasmid and light-chain arm TC253 plasmid were co-transfected into HEK293 cells, and the encoded protein TC350-253 were purified from the cell supernatant using nickel and ceramic HA affinity chromatography. The purified protein (2 µg/lane) were analyzed by SDS-PAGE under reducing (R) or non-reducing (NR) condition. The molecular weight of this bispecific antibody is about 75 kD, and the purity is great than 90% by two steps purification process.

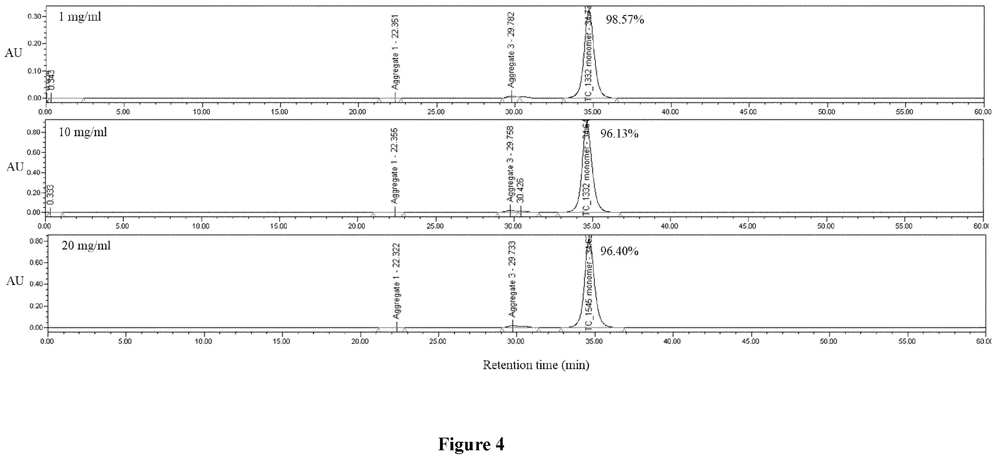

Figure 4 shows the SEC-HPLC analysis of purified TC1545 at three different concentration. The purified TC1545 fusion protein in DPBS + 0.1 M NaCl at pH 7.4 was concentrated with Amicon^{®} Ultra Centrifugal Filter, 30 kDa (UFC903096, Merck Millipore) to 1 mg/mL, 10 mg/mL or 20 mg/mL, respectively, and filtered by Spin-X^{®} Centrifuge Tube Filters. 20 µg of purified protein were analyzed with SEC-HPLC. High-performance liquid chromatography (HPLC) was performed on a Waters Alliance 2695/2996 system equipped with an auto-sampler and a variable wavelength UV detector, using the size-exclusion TSKgel (TOSOH) G3000SW.
Figure 5 shows the SEC-HPLC analysis of purified TC350-253 at 1 mg/mL, 10 mg/mL, and 20 mg/mL three different concentration.
Figure 6 shows the DSC thermogram of TC1545 and TC350-253 analyzed with Malvern, Auto PEAQ-DSC.
Figure 7 shows the SEC-HPLC analysis of purified TC1545 performing the stability for storage at 4°C for 0, 3, 6, 9 and 12 months.
Figure 8 shows the SEC-HPLC analysis of purified TC1545 performing the acceleration stability at 40°C for a month.
Figure 9 shows the binding activity of the TC1545 protein against recombinant human ANG2 in indirect ELISA assays.
Figure 10 shows the binding activity of the TC1545 protein against VEGF-A in indirect ELISA assays.
Figure 11 shows the surface plasmon resonance (SPR) sensorgrams at 7 different concentrations of TC1545 toward recombinant human ANG2, conducted with a Biacore 8K SPR system (Cytiva).
Figure 12 shows the surface plasmon resonance (SPR) sensorgrams at 7 different concentrations of TC1545 toward recombinant human VEGF-A, conducted with a Biacore 8K SPR system.
Figure 13 shows the dual antigen binding activity of TC1545 on ANG2 and VEGF-A by Bio-Layer Interferometry system (BLI).
Figure 14 shows an inhibitory effect of the bi-functional fusion protein TC1545 on the total mesh area of HUVEC cells tube formation induced by recombinant VEGF-A and ANG2.
Figure 15 shows an inhibitory effect of the bi-functional fusion protein TC1545 on the total of HUVEC cells tube length formation induced by recombinant VEGF-A and ANG2.
Figure 16 shows an inhibitory effect of the bi-functional fusion protein TC1545 and other reference antibodies on HUVEC cells proliferation induced by recombinant VEGF-A.
Figure 17 shows the result of TC1545 and Faricimab binding activity against ANG2 mutants. ANG2 mutants were pre-coated on the wells and incubated with constant concentration of purified TC1545 or recombinant Faricimab. The bound proteins were then detected with HRP conjugated goat anti-human Fab specific antibody, and OD₄₅₀ absorbance value were determined.
Figure 18A-18B show the backbone structure models of TC1545/ANG2 and Faricimab/ANG2 complex generated by AlphaFold2. The bottom part is ANG2 molecule and the upper part is Fab of TC1545 (Figure 18A) or Faricimab (Figure 18B), respectively. The side chains of four residues (K412, D448, F469, and Y475) located on the interface and mutated to Ala for epitope mapping by ELISA binding, were shown in sticks models.

### DETAILED DESCRIPTION OF THE INVENTION

Unless otherwise defined herein, scientific and technical terms used herein have the meanings that are commonly understood by those of ordinary skill in the art.

As used herein, the term "antibody" is a protein consisting of one or more polypeptides substantially encoded by immunoglobulin genes. A typical antibody is a tetramer that is composed of two identical pairs of polypeptide chains, each pair having one "light" and one "heavy" chain.

The term "antibody variable region" as used herein refers to an N-terminal region of an antibody variable chain comprising amino acid residues that are primarily responsible for antigen recognition. Those of ordinary skill in the art are readily able to identify an antibody variable region and to determine the minimum size needed to confer antigen recognition. The antibody variable region may be a single chain antibody, for example a single chain fragment variable (scFv) antibody in which a variable heavy chain region and a variable light chain region are joined together (directly or through a peptide linker) to form a continuous polypeptide. The scFv antibody may be chemically synthesized or may be expressed from a nucleic acid including V_{H}- and V_{L}-encoding sequences either joined directly or joined by a peptide-encoding linker.

As used therein, the term "subject" includes any human or nonhuman animal. The term "nonhuman animal" includes, but is not limited to, vertebrates such as nonhuman primates, sheep, dogs, cats, rabbits and ferrets, rodents such as mice, rats and guinea pigs, avian species such as chickens, amphibians, and reptiles. In preferred embodiments, the subject is a mammal such as a nonhuman primate, sheep, dog, cat, rabbit, ferret or rodent. In more preferred embodiments, the subject is a human. The terms, "subject," "patient" and "individual" are used interchangeably herein.

The present invention provides a bi-functional fusion protein TC1545 that inhibits an angiopoietin (ANG)-TIE signaling pathway and a vascular endothelial growth factor (VEGF)-vascular endothelial growth factor receptor (VEGFR) signaling pathway. In the invention, the fusion protein TC1545 contains an ANG inhibiting domain and a VEGF inhibiting domain thereby providing a significantly improved efficacy in inhibition of angiogenesis through blocking the interactions between ANG, VEGF, or both with their receptors. The fusion protein TC1545 comprises an antigen-binding (Fab) fragment and a single chain fragment variable (scFv) which are fused with a linker. The antigen-binding (Fab) fragment refers to an ANG2 binding motif, containing the Fd fragment (the variable domain and the first constant domain) of heavy chain and the whole light chain of antibody, and the scFv antibody refers to a VEGF binding motif-containing fragment.

Since VEGF and ANG2 were upregulated in a variety of cancer cell types, blocking the ANG-TIE interaction together with/without VEGF blocker, a bi-functional fusion against ANG2 and VEGF is created for treatment of cancers and age-related macular degeneration (AMD) disease.

In the invention, the fusion protein TC1545, which has a light chain arm having the amino acid sequence set forth in SEQ ID NO: 4 and a heavy chain arm having the amino acid sequence set forth in SEQ ID NO: 7, was confirmed to exhibit a higher monomer purity as compared to the fusion protein TC350-253 , which comprises the amino acid sequence set forth in SEQ ID NO: 4 and SEQ ID NO: 8. In addition, the amino acid sequence of heavy chain arm of TC1545 (SEQ ID NO: 7) with unexpected modifications to the heavy chain arm of TC350-253 (SEQ ID NO: 8), particularly a five amino acid residues (DKTHT) insertion between residue 229-230, a two amino acid replacement at residue 289 (Gly) and 483 (Gln) with cysteine residues to form a disulfide-bond, and a six amino acid deletion of residue 491-496 of TC350.

In the invention, an angiogenesis-associated disease refers to age-related macular degeneration (AMD) disease, primary cancer or metastatic cancer. The AMD is an eye disease that may get worse over time, leading to serious, permanent vision loss in people over 50 years old. Normally, AMD affects the macula, an oval-shaped pigmented area in the center of the retina of human eye. On the other hand, the primary cancer and metastatic cancers have various types, comprising carcinomas of breast, colon, rectum, lung, oropharynx, hypopharynx, esophagus, stomach, pancreas, liver, gallbladder and bile ducts, small intestine, urinary tract (including kidney, bladder and urothelium), female genital tract (including cervix, uterus, ovaries, choriocarcinoma, and gestational trophoblastic disease), male genital tract (including prostate, seminal vesicles, testes, and germ cell tumors), endocrine glands (including the thyroid, adrenal, and pituitary glands), skin, hemangiomas, melanomas, sarcomas (including those arising from bone, soft tissues, and Kaposi's sarcoma), tumors of the brain, nerves, eyes, and meninges (including astrocytomas, gliomas, glioblastomas, retinoblastomas, neuromas, neuroblastomas, Schwannomas, and meningiomas), tumors arising from hematopoietic malignancies, acute leukemia, acute lymphoblastic leukemia (ALL), acute myeloid leukemia (AML), B cell lymphoma, Burkitt's lymphoma, chronic myelocytic leukemia (CML), chronic lymphocytic leukemia (CLL), hairy cell leukemia, Hodgkin's and non-Hodgkin's lymphomas, hematopoietic malignancies, Kaposi's sarcoma, malignant lymphoma, malignant histiocytosis, malignant melanoma, multiple myeloma, paraneoplastic syndrome/hypercalcemia of malignancy, or solid tumors.

According to the present invention, the bi-functional protein TC1545 was constituted with two polypeptide chains, one heavy chain arm and one light chain arm. The heavy chain arm of TC1545 (SEQ ID NO: 7) was constructed to have the N-terminal of anti-ANG2 heavy chain Fd fragment (SEQ ID NO: 3), which comprises a variable heavy chain domain (V_{H}) (SEQ ID NO: 1) and the first constant domain of human IgG1 (CH1), and a disulfide-bond stabilized anti-VEGF scFv (SEQ ID NO: 6) of Lucentis. The Fd fragment is fused with the scFv by a linker (SEQ ID NO: 5). The light chain arm of TC1545 contains the anti-ANG2 antibody light chain (LC) fragment (SEQ ID NO: 4), which comprises a variable light chain region (V_{L}) having the amino acid sequence set forth in SEQ ID NO: 2 and a constant domain of light cahin (C_{L}).

In the present invention, the bi-functional protein TC1545 provides a beneficial effect on VEGF and/or ANG2-induced angiogenesis-associated phenotypes. Particularly, it has been confirmed that the bi-functional protein TC1545 provides an unexpectedly improved efficacy in laser-induced choroidal neovascularization, which can be used extensively in the exudative form of age-related macular degeneration (AMD).

As used herein, the term "fusion protein" refers to a protein created through the connection of two or more binding proteins, or motifs, or peptides/amino acid fragments coding for different genes, the translation of which genes results in a single or multiple polypeptides with multi-functional properties derived from each of the original proteins. A fusion protein can include a protein conjugated to an antibody, an antibody conjugated to a different antibody, or an antibody conjugated to a Fab fragment.

As used herein, the term "linker" refers to an amino acid residue or fragment, or a polypeptide comprising two or more amino acid residues joined by peptide bonds that are used to link two peptides, polypeptides or proteins. According to the example of the present invention, the linker is G(G4S)3 linker having amino acid sequence set forth in SEQ ID NO: 5.

As used herein, the term "pharmaceutical composition" refers to a composition or a formulation comprising the fusion protein according to the invention, which can be prepared by mixing the fusion protein according to the invention with optional pharmaceutically acceptable carriers, including solvents, dispersion media, isotonic agents and the like. The carrier can be liquid, semi-solid or solid carriers. In some embodiments, carriers may be water, saline solutions or other buffers (such as serum albumin and gelatin), carbohydrates (such as monosaccharides, disaccharides, and other carbohydrates including glucose, sucrose, trehalose, mannose, mannitol, sorbitol, or dextrins), gel, lipids, liposomes, resins, porous matrices, binders, fillers, coatings, stabilizers, preservatives, antioxidants (including ascorbic acid and methionine), chelating agents (such as EDTA), salt forming counter-ions (such as sodium), nonionic surfactants [such as TWEEN^{™}, PLURONICS^{™} or polyethylene glycol (PEG)], or combinations thereof.

The pharmaceutical composition further comprises one additional therapeutic agent for preventing or treating said disease or disorder to be treated. For example, the additional agent can be a therapeutic agent, a chemotherapeutic agent, an imaging agent, a cytotoxic agent, an angiogenesis inhibitor, a kinase inhibitor, a co-stimulation molecule blocker (comprising anti-B7.1, anti-B7.2, CTLA4-Ig, anti-CD20), an adhesion molecule blocker (comprising anti-LFA-1 antibody, an anti-E/L selectin antibody, a small molecule inhibitor), and an anti-cytokine antibody or functional fragment thereof (comprising an anti-IL-18, an anti-TNF, and an anti-IL-6/cytokine receptor antibody).

The invention is further illustrated by the following examples, which should not be construed as further limiting.

### Examples

### Example 1 Protein Purity Evaluation of TC1545 and TC350-253 by SEC-HPLC After Purification and Concentration

### Bi-Functional Protein TC1545

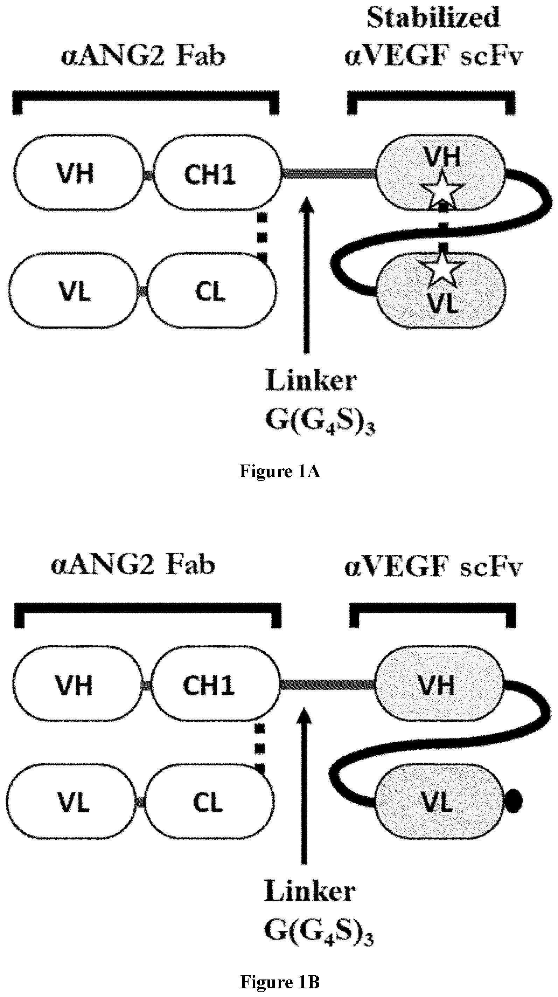

For over-expression of bi-functional protein TC1545, both cDNAs encoded for the light-chain arm and the heavy-chain arm polypeptide of TC1545 leading with a signal peptide for protein secretion in mammalian expression system were sequentially constructed into UCOE-GS vector (Merck) and then over-expressed with CHOZN cell line (Merck). The light chain arm cDNA contains the full light chain of anti-ANG2 clone. The heavy chain arm cDNA contains the Fd fragment (the variable domain plus the first constant domain of anti-ANG2 heavy chain) fused with the disulfide-bond stabilized anti-VEGF scFv (Lucentis VH and VK domains) by a GS linker. Two spatial-nearby residues, 289 (Gly) and 483 (Gln) of its parental heavy chain arm TC350 (SEQ ID NO: 8) within anti-VEGF scFv domain, were mutated with cysteine residues based on its 3D structure information. These 2 cysteine mutations created an intra-disulfide bond within scFv and stabilized the structure of the scFv domain and the full bi-functional fusion protein TC1545 (Figure 1A). The signal peptide at the N-terminal for the secretion of soluble fusion proteins for both heavy chain arm and light chain arm were experimentally selected from a panel of tested signal peptides. The stable pool of protein expression CHOZEN GS -/- cell line was established after transfected with bi-functional fusion TC1545/UCOE-GS vector, using glutamine-based metabolic selection. The over-expressed TC1545 was purified from cell culture supernatant via Mabselect VL resin, ceramic HA affinity chromatography and ionic exchange chromatography, and then concentrated with Ultrafiltration and Diafiltration (UF/DF) (Pall Corporation) system. The purified protein (2 µg / lane) was analyzed by SDS-PAGE under reducing or non-reducing condition (Figure 2). Lane R refers to reduced condition; Lane NR refers to non-reduced condition. The results indicated that TC1545 bi-functional protein was properly expressed with a molecular weight about 75 kD, and greater than 90 % purity was obtained.

To check the characteristic of TC1545 at high concentration to meet administrated dose used in the ophthalmic disease, the purity and stability of TC1545 was measured by SEC-HPLC analysis at three different concentrations of 1 mg/mL, 10 mg/mL and 20 mg/mL, respectively. As shown in Figure 4, high-performance liquid chromatography (HPLC) was performed on Waters Alliance 2695/2996 system equipped with an auto-sampler and a variable wavelength UV detector. The purities of TC1545 at 1, 10, and 20 mg/mL are 98.57%, 96.13%, and 96.40%, respectively.

The results indicated that TC1545 is stable at high concentration. There is only 2% decrease of purity when the concentration of TC1545 was raised from 1 mg/mL to 20 mg/mL.

### Bi-Functional Protein TC350-253

For over-expression of bi-functional protein TC350-253, cDNAs encoded for the light-chain arm (TC253) and the heavy-chain arm (TC350) polypeptide were separately constructed to pCI vector- a neo expression vector under the control of a CMV promoter leading with a signal peptide for protein secretion in mammalian expression system. The light chain arm cDNA contains the full light chain of anti-ANG2 clone. As shown in Figure 1B the heavy chain arm cDNA contains the Fd fragment (the variable domain plus the first constant domain of anti-ANG2 heavy chain) fused with the anti-VEGF scFv (Lucentis VH and VK domains) by a GS linker.

For over-expression of TC350-253 fusion protein, both heavy-chain arm plasmid TC350 and light-chain arm plasmid TC253 were co-transfected into HEK293 cells. TC350-253 fusion protein was transiently expressed by HEK293 cells and purified from the transfected cell culture supernatant via nickel and ceramic HA affinity chromatography. The purified protein (2 µg / lane) was analyzed by SDS-PAGE under reducing or non-reducing condition (Figure 3). The TC350-253 bi-functional protein was properly expressed with a molecular weight about 75 kDa, and about 90 % purity was obtained.

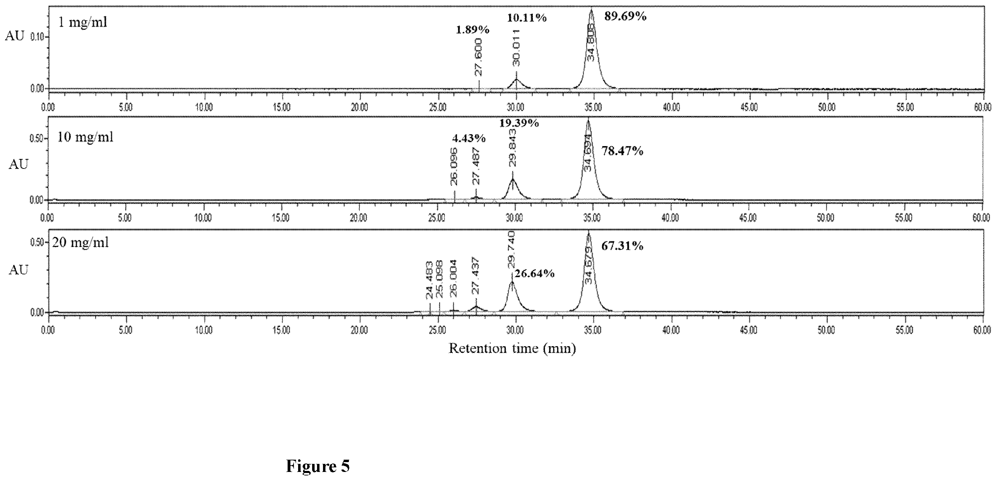

To compare the stability of the TC350-253 with its derived counterpart TC1545 with disulfide-stabilized anti-VEGF scFv, the stability and purity of TC350-253 fusion protein was measured by SEC-HPLC analysis at three different concentrations of 1 mg/mL, 10 mg/mL and 20 mg/mL, respectively. As shown in Figure 5, the percentage of TC350-253 main peak gradually declined from 89.69% , 78.47%, and 67.31% , respectively, when the concentration of the bi-functional protein at concentration of 1 mg/mL, 10 mg/mL, and 20 mg/mL. On other hand, the percentage of high molecular weight peak was gradually increased from 10.11%, 19.39%, to 26.64% accompanied by rising protein concentration from 1 mg/mL, 10 mg/mL, to 20 mg/mL.

The result indicated that bi-functional protein TC350-253 is unstable at high concentration and cannot meet the expected concentration for ophthalmic disease treatment.

As shown in Table 1, the SEC-HPLC analysis results indicated that TC1545 has a higher monomer purity than TC350-253 at the concentration of 1 mg/ml, 10 mg/ml and 20 mg/ml, the stability of TC1545 was improved through the changes of primary amino acid sequence of TC350-253, particularly introducing a disulfide-bond between the VH and VL of anti-VEGF scFv. The disulfide-bond to stabilize the anti-VEGF scFv of TC1545 can improve the protein stability at high concentration.

**Table 1. Summary of SEC-HPLC analysis result of TC350-253 and TC1545**

| Bi-specific fusion protein concentration | Monomer purity (%) | |
|---|---|---|
| | TC1545 | TC350-253 |
| 1 mg/ml | 98.57 | 89.69 |
| 10 mg/ml | 96.13 | 78.47 |
| 20 mg/ml | 96.40 | 67.31 |

### Example 2 DSC Analysis of TC1545

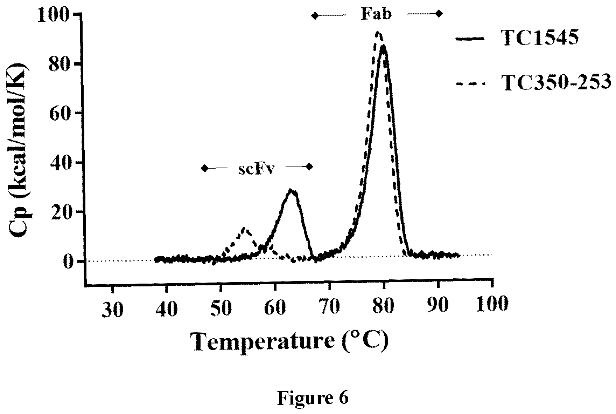

To clarify the biophysical properties of bifunctional fusion protein, both the purified TC1545 and TC350-253 were analyzed by a differential scanning calorimeter (DSC) (Malvern, Auto PEAQ-DSC). The results of two major melting temperature peaks for both TC1545 and TC350-253 were summarized at Table 2. As shown in Figure 6, the first transition temperature (indicated as Tm₁) corresponds to the unfolding anti-VEGF scFv domain, and the second transition temperature (indicated as Tm₂) corresponds to the unfolding of the anti-ANG2 Fab domain. For TC1545, the Tm1 of scFv domain is 63.09 °C and Tm2 of Fab domain is 80.67 °C, respectively. For TC350-253, the Tm1 of scFv domain is 54.34 °C and Tm2 of Fab domain is 79.88 °C, respectively. The thermal stability of scFv domain of TC1545 is higher than that of TC350-253, most likely can be attributed to the disulfide bond between the VH and VL regions of the anti-VEGF scFv domain.

**Table 2. Summary of Tm (°C) values of TC350-253 and TC1545.**

| BsAb (Fab-scFv) | **TC1545** | **TC350-253** |
|---|---|---|
| Tm₁ (°C) | 63.09 | 54.34 |
| Tm₂ (°C) | 80.67 | 79.88 |

### Example 3 Stability Analysis of TC1545 Storage by SEC-HPLC

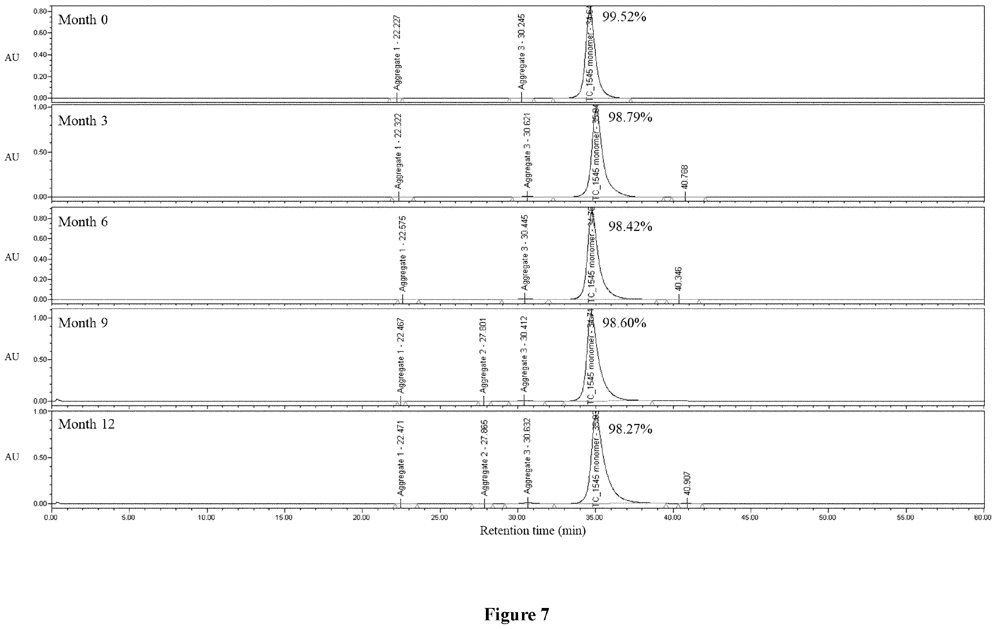

To meet the targeted concentration as a drug substance for ophthalmic disease treatment, the bifunctional fusion protein TC1545 was concentrated up to 200 mg/mL in a buffer containing 20 mM Histidine-HCl and 50 mM NaCl at pH 7.0. The purities of the concentrated TC1545 and the samples storage at 4 °C for 3, 6, 9 and 12 months were analyzed by SEC-HPLC. As shown in Figure 7, the purity of original sample is 99.52%; and after storage at 4°C for 3, 6, 9, and 12 months, the purity is 98.79%, 98.42%, 98.60%, and 98.27%, respectively. The purity of concentrated TC1545 at 200 mg/mL in our formulation almost did not changed. The results indicated that the new version bi-functional fusion protein TC1545 in our formulation is stable for storage at least 1 year without loss its purity.

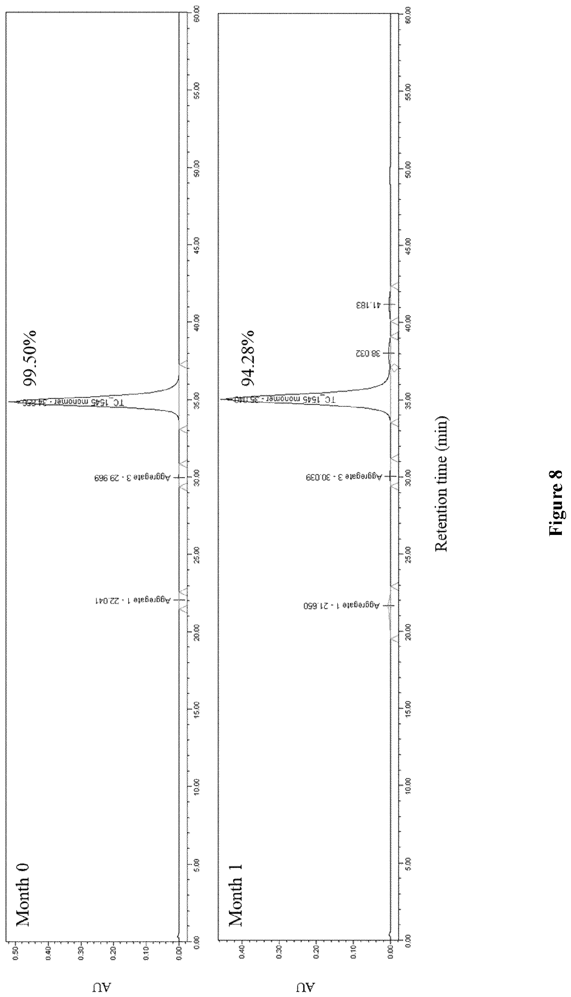

As shown in Figure 8, the original purity of TC1545 is 99.50%, and decreased to 94.28% after acceleration stability study at of was performed by SEC-HPLC analysis. The monomer purity of TC1545 remained at 94.28% after 40°C for one month.

### Example 4 In Vitro Binding of Bi-functional Angiogenesis Blocker by ELISA

To evaluate the binding affinity of bi-functional TC1545 protein, ELISA binding assay was conducted against with ANG2 and VEGF-A antigens. ANG2 (PeproTech Inc.) or VEGF-A (PeproTech, Inc.) were coated (100 ng/well) on the wells for overnight, and then washed with 1X phosphate buffered saline (PBS) and blocked with 400 µL 5 % non - fat skin milk in PBS for 1 hr. After blocking, various concentrations of purified TC1545 protein were incubated in the ligand pre-coated wells. 100 µL of 3 - fold serially diluted purified TC1545 started from 30 nM were added to each corresponding well and incubated for 1 hr at room temperature on shaker. After binding, wells were washed 3 times with PBST (1X PBS with 0.1 % Tween 20) before adding 100 µL of 1:2000 diluted goat anti-human Fab HRP conjugates (Jackson ImmunoResearch Inc.) to each well and incubated on shaker for another hour. After final washing, TMB reagent (Thermo Fisher Scientific, Inc.) was added and OD absorption at 450 nm was measured.

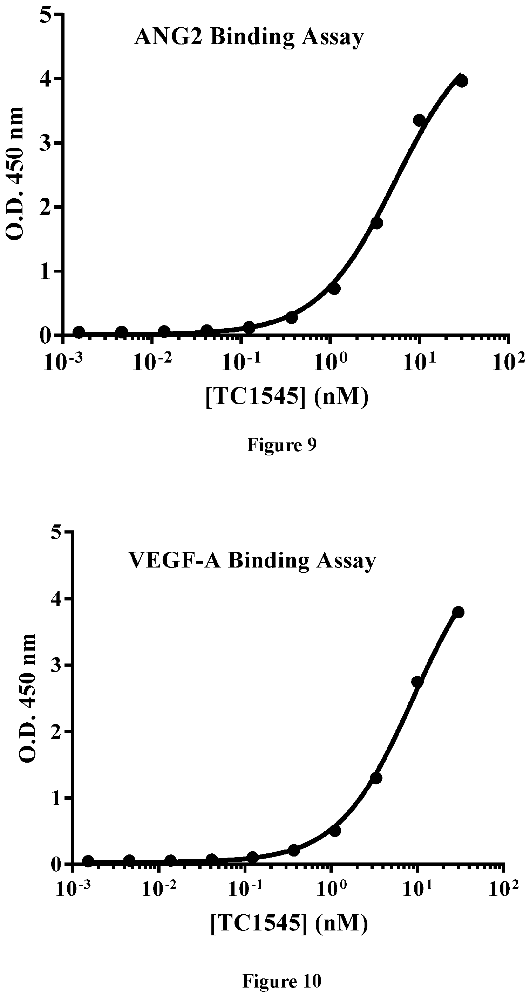

The binding assay was performed in three individual test and the data were analyzed by sigmoidal curve fitting using Prism 6. Binding of TC1545 to ANG2 (Figure 9) or VEGF (Figure 10) were assessed by ELISA assay. In the assay, the TC1545 were able to bind ANG2 and VEGF-A at EC₅₀ of 5.408 nM and 8.986 nM, respectively, and confirmed this fusion bifunctional protein can bind with both ANG2 and VEGF-A to have potential bi-functions to mediate to the ANG - TIE and VEGF - VEGFR pathways through the inhibition of ligand binding and receptor activation.

### Example 5 In Vitro Affinity Assessment Assay of Bi-Functional Angiogenesis Blockers by SPR analysis

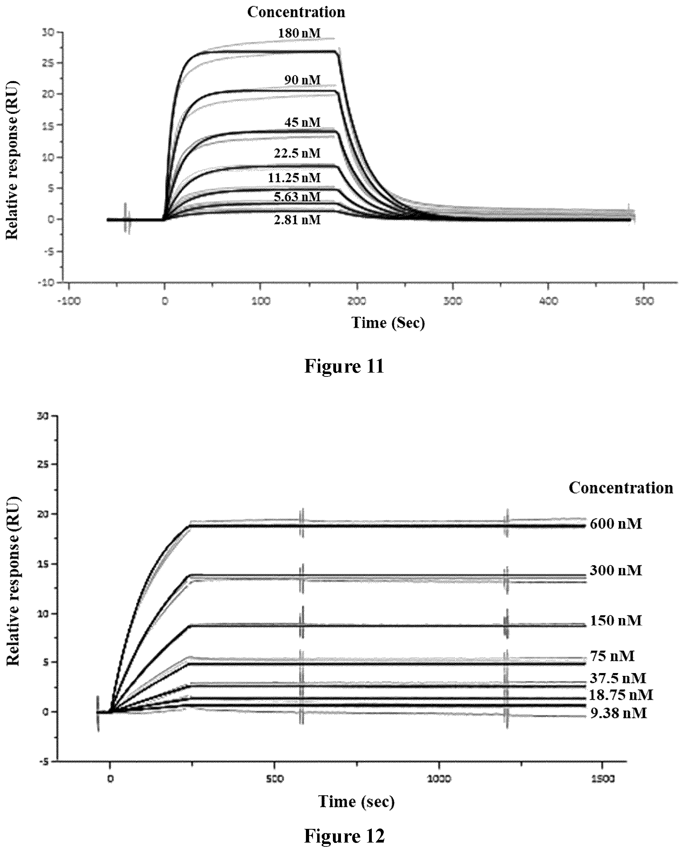

Binding affinity (*K_{D}*) of TC1545 toward ANG2 and VEGF-A was performed by surface plasmon resonance experiments with a Biacore 8k instrument (Cytiva, Inc.) as described below. For ANG2 binding kinetic analysis, ANG2 (PeproTech, Inc.) containing a His tag at C-terminal was captured by an anti-histidine antibody immobilized on the CM5 chip. All assays were performed with a running PBSP buffer containing 10 mM phosphate buffer with 2.7 mM KCl, 137 mM NaCl (diluted from Cytiva 10X PBS Buffer) and 0.01% v/v Surfactant P20 (Cytiva, cat. BR100054)) at 25 °C. A range of TC1545 concentration was prepared in a two-fold serial dilution started from 180 nM to 2.81 nM in running buffer (PBS with 0.005% v/v Surfactant P20 (Cytiva, Inc.) to pass over the surface of captured ANG2 sequentially at a flow rate of 10 µL/min for 180 s. Then, TC1545 were replaced in running buffer for 300 s for disassociation. The final sensorgram was obtained and *K_{D}* values were calculated from all binding curves based on their global fit into a two-state binding model by Biacore 8k data analysis software (Figure 11).

The results showed that TC1545 binds to ANG2 with *K_{D}* of 7.78 x 10⁻⁸ M, with a moderate fast *on-rate* with *kₐ =* 4.51 x 10⁵ (M⁻¹xS⁻¹) and a relatively fast off-rate with *k*_{d} = 3.56 x 10⁻² S⁻¹.

For VEGF-A binding assessment, VEGF-A₁₆₅ protein (PeproTech, cat. 100-20) was diluted to 30 nM with 10 mM sodium acetate, pH 5.5 (Cytiva, cat. BR100352), and then immobilized onto the surface of a Series S CM5 chip (Cytiva, cat. BR100530) to about 180 RU. TC1545 protein was performed in a two-fold dilution series from 600 nM to 9.38 nM, at a flow rate of 50 µl/min using a multi-cycle kinetics program with an association time of 240 second and a dissociation time of 1200 second. Running buffer was also injected using the same program for background subtraction. At the end of the dissociation phase of each concentration groups, inject one short pulse (60 second) of regeneration buffer (10 mM Glycine at pH 2.5) (Cytiva, cat. BR100356) to remove any non-covalently bound protein on the surface of a CM5 chip. Affinity constant *K_{D}* values (derived from *k_{off}* / *kₒₙ*) were calculated from all the binding curves based on their global fit to a 1:1 binding model by Biacore 8K data analysis software (Figure 12).

The results showed that TC1545 binds to VEGF-A₁₆₅ protein with *K_{D}* of 8.24 x 10⁻¹² M, with *on-rate kₐ =* 1.42 x 10⁴ (M⁻¹xS⁻¹), and a very slow *off-rate* with *k_{d} =* 1.12 x 10⁻⁷ S⁻¹. The binding affinity of anti-VEGF scFv domain with VEGF of TC1545 was comparable with the published data of anti-VEGF antibody Lucentis with *K_{D}* of 3.5 x 10⁻¹² (Regula, J. T., et al. Targeting key angiogenic pathways with a bispecific CrossMAb optimized for neovascular eye diseases. EMBO molecular medicine, 8(11), 1265-1288.).

### Example 6 In Vitro Dual Antigen Binding Activity of TC1545 by BLI

In the previous ELISA data, the bi-functional antibody TC1545 was demonstrated can bind with ANG2 and VEGF-A, respectively. To study whether TC1545 was able to bind with ANG2 and VEGF-A simultaneously, three different in-tandem protocols were conducted for TC1545 binning assay by using the Octet^{®} Red96 system (ForteBio, Menlo Park, CA). After coating TC1545 onto FAB2G biosensor, the first protocol loaded ANG2 as first antigen and then loaded VEGF-A as second antigen. The second protocol loaded the VEGF-A first and then ANG2; and the third protocol simultaneously loaded both ANG2 and VEGF-A.

In the experiment, FAB2G biosensors (ForteBio, Menlo Park, CA) were soaked in PBS buffer for 20 mins before the binding assay, and all steps were performed at 30 °C with an agitation speed of 1,000 rpm on the flat bottom 96-well black plate. TC1545 was first captured onto FAB2G biosensors for 190 seconds to obtain enough wavelength shift, followed by a baseline step in PBS buffer for 60 seconds. Then, the FAB2G biosensor was dipped into well with 300 nM of first antigen (ANG2 or VEGF-A) in PBS buffer for 300 seconds, and then dipped into well with 300 nM of second antigen (VEGF-A or ANG2) for 300 seconds. For the third protocol, both antigens ANG2 and VEGF-A were mix together in the well as second antigen. The binding response of TC1545 and ligands was measured as the change in the thickness of the biological layers, and was correlated to the number of molecules bound to the tip surface of the biosensor. The wavelength shift was directed recorded and displayed on a sensogram, and data from BLI experiments was analyzed by Octet Data Acquisition software.

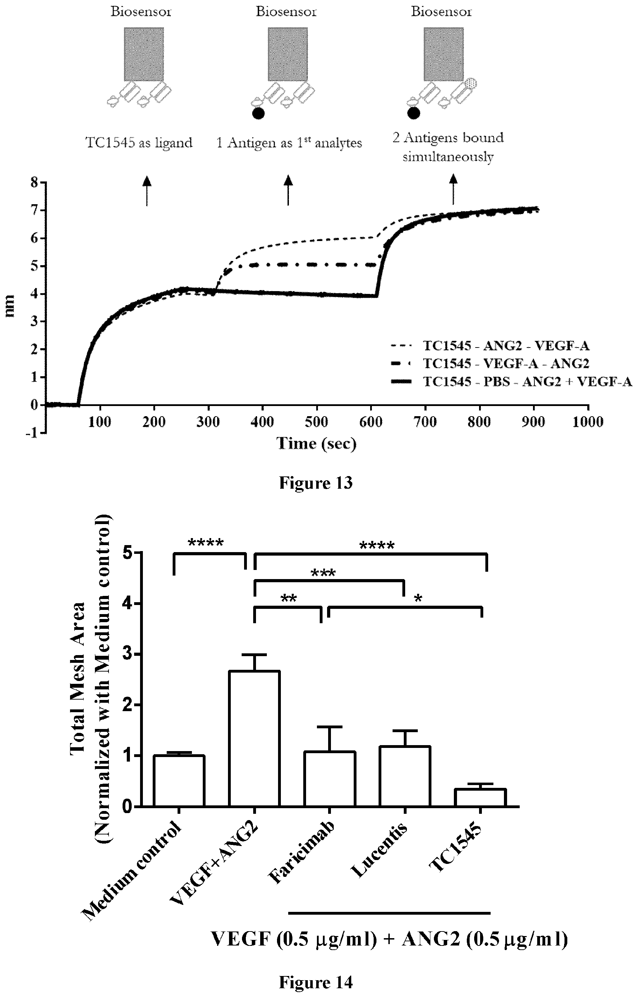

As shown in Figure 13, the wavelength shifts reached to 4 nm after TC1545 was captured, and showed further change when first analytes (ANG2 or VEGF-A) were loaded onto the biosensor, except PBS buffer only. Finally, all three individual conditions exhibited the similar interference pattern which the wavelength shifts were about 7 nm after the biosensors were soaked with 2^{nd} antigens, VEGF-A, or ANG2, or ANG2 plus with VEGF-A. This result indicated that the bi-functional antibody TC1545 was able to bind with ANG2 and VEGF-A independently, no matter which one bind with first, or bind simultaneously. It is ascertained that TC1545 was able to modulate both angiogenesis signaling of VEGF-VEGFR and TIE-ANG2 pathway.

### Example 7 Bi-Functional Protein TC1545 Inhibited VEGF and ANG2- Induced HUVEC Cells Tube Formation

*In vitro* Matrigel tube formation assay in human umbilical vein endothelial cells (HUVEC cells, Lonza, USA) was used to evaluate the function of bi-functional angiogenesis blocker TC1545. HUVEC cells were incubated in Medium 199 without serum for 2 hrs, and then trypsinized by Accutase (Thermo Fisher Scientific). 4x10³ HUVEC cells were seeded onto Matrigel pre-coated wells containing VEGF (0.5 µg / mL) and ANG2 (0.5 µg / mL) with 0.67 µM of TC1545 or recombinant Faricimab (Thermo Fisher Scientific) or recombinant Lucentis at 37 °C for 4.5 hrs. Recombinant Faricimab and Lucentis were used as positive control to assess the assay validity in the experiment. Quantification of endothelial network formation was performed by Image J angiogenesis system (5 images/sample). The samples were represented as fold change as compared to medium control. HUVEC cells formed a primary vascular tubular network under VEGF and ANG2 treatment for 4.5 hrs on Matrigel.

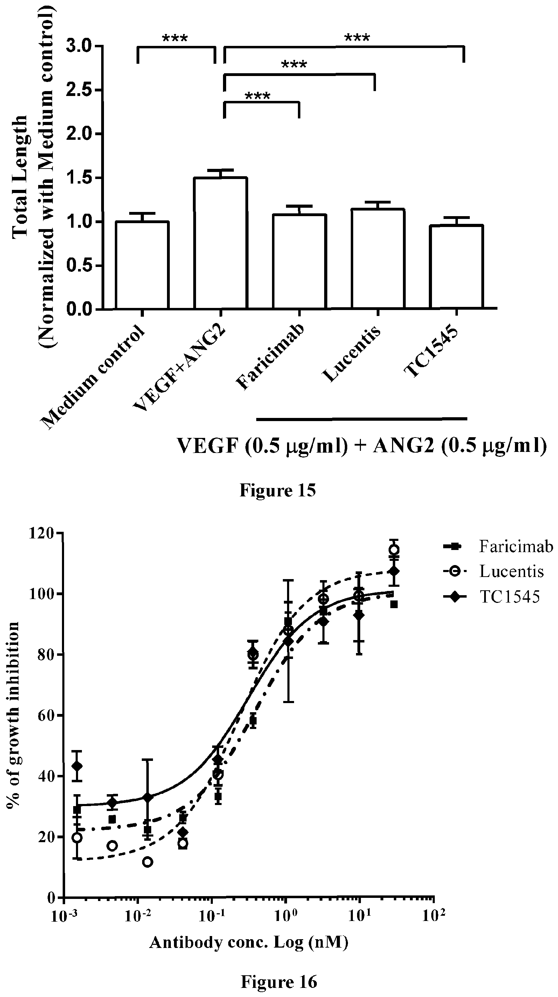

According to the results, the bi-functional angiogenesis blocker TC1545 significantly disrupted the formation of tubular structures on total mesh area (as shown in Figure 14) and total length measurement (as shown in Figure 15). The bioactivity of TC1545 to inhibit the HUVEC tube formation was better than recombinant Faricimab or Lucentis, particularly for the mesh area.

### Example 8 Bi-Functional Protein TC1545 Inhibited VEGF-Induced HUVEC Cells Proliferation

To demonstrate the blocking of VEGF pathway, the inhibition activity of TC1545 on the VEGF-induced HUVEC cells proliferation was conducted. In the experiment, 100 µL of 8 ng/mL VEGF (R&D systems, Inc.) was incubated with various concentrations of TC1545, recombinant Faricimab (Thermo Fisher Scientific), or recombinant Lucentis at room temperature with light protection for overnight. HUVEC cells were seeded in Gelatin-coated 96-well plate at the density of 3500 cells per well with 100 uL growth medium. Then, the pre-incubated mixtures were transferred into individual well containing with HUVECs. After 72 hrs incubation at 37 °C with 5 % CO₂, cell growth was assayed by adding 10 uL MTS detection reagent (Promega, USA) to each well for 2.5 hrs and measured OD absorption at 490 nm by ELSIA reader.

As shown in Figure 16, the bi-functional fusion proteins TC1545 exhibited comparable abilities with Faricimab and Lucentis for inhibiting VEGF-induced HUVEC proliferation, with IC₅₀ of 0.3197 nM, 0.3944 nM, and 0.2481 nM respectively.

### Example 9 Prediction of ANG2 binding epitope with TC1545 by AlphaFold and evaluation by point mutation binding assay

To identify the binding epitope of ANG2 in complex with TC1545, AlphaFold, an AI system for protein 3D structure and protein-protein interaction prediction, was used to model TC1545 Fab and ANG2 complex. Based on the conserved structure models generated by AlphaFold, the interface was visualized and analyzed by structure viewer PyMol program. The binding epitope of ANG2 for TC1545 was overlap with the ANG2-TIE2 interface. To compare with bench marker anti-ANG2/anti-VEGF bispecific antibody Faricimab, the structure model of Faricimab in complex with ANG2 was also predicted by AlphaFold, and its binding epitope was also overlap with the ANG2-TIE2 interface, but with slightly different site and orientation comparing with those of TC1545-ANG2 complex. The prediction of ANG2 epitope for TC1545 and Faricimab was confirmed since both TC1545 and Faricimab can block ANG2 to bind with its receptor TIE2 in solid phase ELISA and cellular binding assay on TIE2-overexpressed cell line (data not shown).

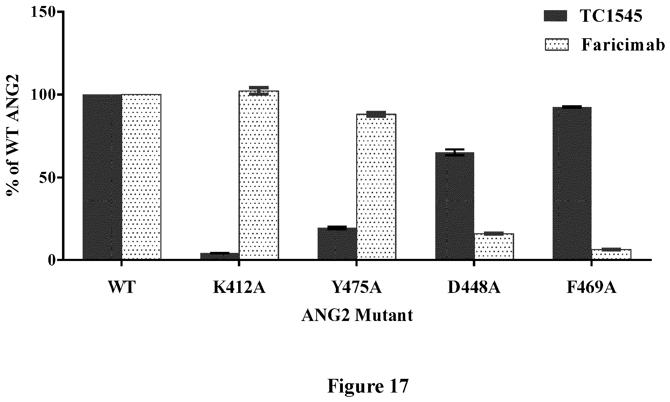

To evaluate the binding residues of ANG2 in complex with TC1545 and Faricimab, various ANG2 Ala mutants were created by site-directed mutagenesis method based-on the calculation of protein-protein binding free energies, and over-expressed in mammalian expiCHO cells. The ELISA binding assay of TC1545 and Faricimab against with various ANG2 mutants was then conducted. Various ANG2 mutants (2 ug/mL) were coated on the wells and then incubated with constant concentration of purified TC1545 (at 30 nM) or recombinant Faricimab (at 3 nM) to obtain comparable ELISA readout signal. The bound proteins were then detected with HRP conjugated goat anti-human Fab specific antibody, and OD450 absorbance value were measured by ELISA reader. As shown in Figure 17, among these ANG2 mutants, four crucial epitope residues (K412, D448, F469, and Y475) of ANG2 were identify from their ELISA data.

The ELISA binding assay showed that TC1545 almost lost its binding ability to K412A and Y475A mutants of ANG2, but Faricimab still kept high binding activity to these mutants. In contrast, D448A and F469A mutants of ANG2 affected the binding activity for Faricimab, but did not affect for TC1545. It revealed that the crucial binding residues of ANG2 epitope for TC1545 and Faricimab are different, even though both can block ANG2-TIE2 interaction in ELISA and cellular binding assay with almost equal potency.

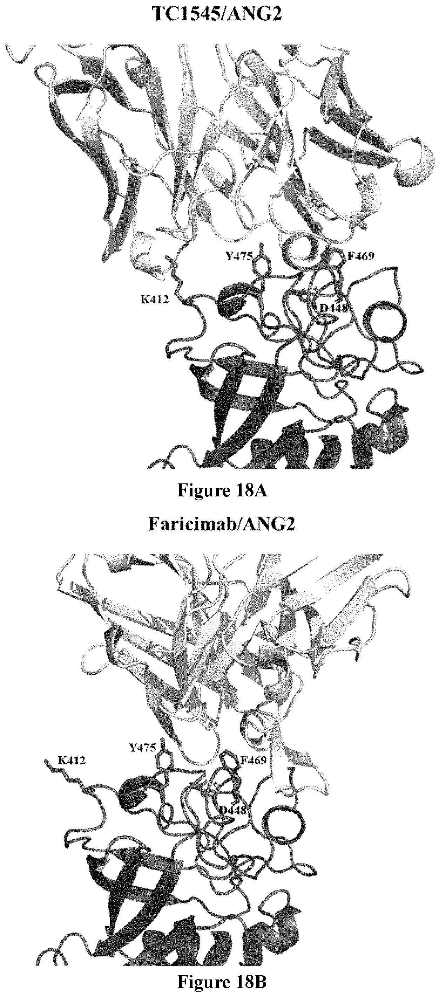

The results evidenced by the TC1545/ANG2 and Faricimab/ANG2 complex structure models predicted by AlphaFold program. Four crucial residues of ANG2 for interaction with TC1545 and Faricimab are shown in stick mode and labelled. The binding epitope of ANG2 for TC1545 is partially overlap with the interface of ANG2-TIE2 receptor crystal complex structure, and residues K412, D448, and Y475 of ANG2 are in contact with antigen binding site formed by six CDRs of TC1545 Fab (Figure 18A). However, residue F469 of ANG2 do not in contact with TC1545 and its mutants showed no effect on binding with TC1545 as shown in Figure 17. For Faricimab/ANG2 complex model, residues D448 and F469 of ANG2 are in contact with the antigen binding site of Faricimab, and residues K412 and Y475 do not in contact with Faricimab (Figure 18B). The complex structure models of TC1545/ANG2 complex and Faricimab/ANG2 complex are consistent with the ELISA results of those residues mutants.

While the present invention has been disclosed by way preferred embodiments, it is not intended to limit the present invention. Any person of ordinary skill in the art may, without departing from the spirit and scope of the present invention, shall be allowed to perform modification and embellishment. Therefore, the scope of protection of the present invention shall be governed by which defined by the claims attached subsequently.

## Claims

1. A bi-functional fusion protein TC1545, which is composed of (1) a light chain arm having the amino acid sequence set forth in SEQ ID NO: 4 and (2) a heavy chain arm having the amino acid sequence set forth in SEQ ID NO: 7, comprising (a) an antigen-binding (Fab) fragment of anti-ANG2 antibody, (b) a stabilized single-chain fragment variable (scFv) fragment of anti-VEGF antibody, and (c) a linker, wherein the light chain arm and the heavy chain arm is bridged with a disulfide bond.

2. The bi-functional fusion protein TC1545 of claim 1, wherein the light chain arm comprises a variable light chain region (VL) having the amino acid sequence set forth in SEQ ID NO: 2.

3. The bi-functional fusion protein TC1545 of claim 1, wherein the heavy chain arm comprises (1) a Fd fragment having the amino acid sequence set forth in SEQ ID NO: 3, (2) a scFv fragment having the amino acid sequence set forth in SEQ ID NO: 6, and (3) a linker having the amino acid sequence set forth in SEQ ID NO: 5.

4. The bi-functional fusion protein TC1545 of claim 1, wherein the antigen-binding (Fab) fragment and the single chain antibody (scFv) fragment are fused with a linker having the amino acid sequence set forth in SEQ ID NO: 5.

5. The bi-functional fusion protein TC1545 of claim 1, which has unexpected modifications to the TC350-253, particularly a five amino acid insertion between residue 229-230, a two amino acid replacement at residue 289 and 483, and a six amino acid deletion of residue 491-496 of TC350 having the amino acid sequence set forth in SEQ ID NO: 8.

6. The bi-functional fusion protein TC1545 of claim 1, wherein the antigen-binding (Fab) fragment is an ANG2 binding motif-containing fragment which is composed of the amino acid sequence set forth in SEQ ID NO: 3 and SEQ ID NO: 4.

7. The bi-functional fusion protein TC1545 of claim 1, wherein the single chain antibody (scFv) fragment is a VEGF binding motif-containing fragment having the amino acid sequence set forth in SEQ ID NO: 6.

8. A pharmaceutical composition for use in inhibiting angiogenesis, comprising the bi-functional fusion protein TC1545 according to claim 1 and a pharmaceutically acceptable carrier.

9. The pharmaceutical composition of claim 8, which is effective for preventing or treating age-related macular degeneration (AMD) disease.

10. The pharmaceutical composition of claim 8, which is effective for treating a primary or a metastatic cancer.

11. A bi-functional fusion protein TC1545 of any of claims 1 - 7 or a pharmaceutical composition of any of claims 8 or 9 for use in preventing or treating an angiogenesis-associated disease in a subject.

12. The method of claim 11, wherein the angiogenesis-associated disease comprises age-related macular degeneration (AMD) disease, primary cancer and metastatic cancer.
